# EUROPEAN PATENT SPECIFICATION

(11) **EP 3 821 833 B1**
(45) Date of publication and mention of the grant of the patent: **13.09.2023**
(21) Application number: 19835034.0
(22) Date of filing: 09.07.2019
(51) Int. Cl.: A61B 17/34, A61B 10/02, A61M 5/32

(54) **DEVICE FOR INTRAOSSEOUS NEEDLE REMOVAL**
VORRICHTUNG ZUR INTRAOSSÄREN NADELENTFERNUNG
DISPOSITIF D'EXTRACTION D'AIGUILLES INTRAOSSEUSES

(30) Priority: 10.07.2018 ES 201830688
(43) Date of publication of application: 19.05.2021
(73) Proprietor: Escuela Universitaria de Enfermeria y Fisioterapia San Juan de Dios. Orden Hospitalaria de San Juan de Dios, 28350 Ciempozuelos Madrid (ES); Universidad Pontificia Comillas, 28015 Madrid (ES)
(72) Inventor: DE LA TORRE MONTERO, Julio César, 28350 Ciempozuelos-Madrid (ES)
(74) Representative: Jiménez Diaz, Rafael
(86) International application number: PCT/ES2019/070479
(87) International publication number: WO 2020/012051

(56) References cited:
- WO-A1-2009/070896
- WO-A2-92/19296
- DE-U1-202004 017 862
- US-A- 4 664 654
- US-A- 4 923 447
- US-A- 5 312 364
- US-A- 5 868 711
- US-A- 5 868 711
- US-A1- 2006 106 363
- US-A1- 2010 152 616
- US-A1- 2016 235 431

## Description

### FIELD OF THE INVENTION

The present invention is comprised in the technical field corresponding to the medical device sector. More specifically, the invention concerns, a device specially designed for the process for the safe removal of intraosseous needles.

### BACKGROUND OF THE INVENTION

When administering or drawing fluids to/from a patient through the intravascular route, whether for medicinal products or blood transfusions, there are two alternatives: administration through the intravenous route or through the intraosseous route. In certain situations, where bone marrow is required to be removed, where the patient has sustained burns, presents edemas, or in emergency situations in patients requiring cardiopulmonary resuscitation, the intraosseous route is a preferred solution for the delivery of liquid solutions to the patient through the intravascular route. In the last mentioned, for use in emergencies, it is key to use elements that protect both the patient and the health care staff carrying out the removal technique, which must be safe.

Once said administration of fluid has been performed, it is necessary to remove the needle from the body of the patient. However, intraosseous injection and/or administration devices are generally not adapted for facilitating the method of removal, which complicates said method and can generate damage, discomfort, or pain for the patient, in addition to being harmful for the medical professional carrying out the removal technique, as they are not used in usual clinical practice, nor are they described in protocols for the removal of tools suitable for that purpose, but rather the removal is performed with an instrument designed for another purpose.

To mitigate the aforementioned problem, there are some products on the market, such as, for example, the "Arrow EZ-IO Needle Set" marketed by the company Teleflex Inc. Said device is based on a syringe with a connection to an intraosseous needle. Once the syringe is connected to the needle, the medical personnel proceeds to manual removal by means of a joint rotation and traction movement on the syringe to remove the implanted needle. A non-specialized device, such as a syringe, is thereby used by way of a handle or gripping part. Given the geometry of the needle and the nature of the forces applied during removal, that is, rotation and traction, maintaining alignment between the needle and the syringe is a complex task which may cause damage to tissues near the needle insertion point or a feeling of discomfort in the patient. Moreover, and given the design of intraosseous needles, the use of a device not specifically designed for the safe removal presents a risk to anti-accidental puncture safety for the professional performing the technique.

Needle insertion devices such as the one described in patent US5868711, which discloses an implantable intraosseous needle assembly and various means for injecting, using, and extracting that assembly into a bone containing bone marrow are also known.

Said document also relates to an extractor comprising an outer positioning element, by way of a sleeve, wherein the intraosseous needle is partially protected in the interior once it has been removed, minimizing the risks of accidental punctures. Protection, however, is not complete, as the distal portion which includes the tip of the needle in the mentioned device continues to protrude through the through hole of its receiver, even once it has been assembled in the conical standoff acting as a connector therein. Therefore, the sleeve disclosed in the extractor assembly of US5868711 is not a protective element, per se, but rather an element for positioning the extractor with respect to the base thereof. Furthermore, the removal operation with this device is preferably performed by means of a successive rotation mechanism, which allows the intraosseous needle to be removed by means of turning a proximal handle. This mechanism therefore causes it to be necessary to rotate the needle multiple times while it is still within the bone or tissue for complete removal, which may damage said bone or tissue during the removal process.

As described in patent application US2010152616, there is also known in the state of the art a device for safely accessing bone marrow and other tissues. Said device comprises a needle assembly, a sensor mechanism, and an actuator for the insertion of the needle in the tissue of a patient. The device also comprises a needle protector which is configured for sliding inside the base when the needles are inserted in the skin, by way of a guide. In this context, said document refers exclusively to devices for the insertion and not the removal of needles, therefore being relative to a technical problem different from the one of the present application.

The present invention proposes an ergonomic device for the removal of intraosseous needles, specially adapted for exerting the traction and rotation forces necessary to remove said needles in a simple and minimally aggressive manner for the patient, in addition to providing safety for the professional carrying out the safe removal technique, when performing removal of the device with a complete and specific biosafety element.

Document US5868711A discloses a device and method for rapid access to the bone marrow for the infusion of drugs and other fluids into the bone marrow; sampling the bone marrow; and monitoring the physical properties of the blood and bone marrow. These operations may proceed sequentially or simultaneously. A "smart" controller may be utilized to direct or control these operations. An implantable intraosseous needle assembly and various means for injecting, utilizing, and extracting that assembly into a bone containing bone marrow are disclosed.

### BRIEF DESCRIPTION OF THE INVENTION

The technical problem solved by the present invention with respect to the prior art mentioned in the preceding section is to provide improved protection features for the user of the device during the operation of removal of the intraosseous needle. This is because given that once the needle has been removed, or even at virtually the same time, depending on the skill of the user, the user may immediately and readily extend a protective element concentric with the needle, along the entire length thereof, preventing any potential damage with the pointed end of said needle. It is further mentioned that in the arrangement of the present invention, easier removal is observed as a rotational movement can be combined with a traction movement, which is not found in the aforementioned prior art documents. The user could perform said traction movement simultaneously with a movement for extending the protective element, thereby combining an enhanced protection for the user handling the device with a faster and cleaner removal operation that is, therefore, less painful for the patient.

Likewise, the present invention provides a device for the removal of needles wherein the of removal operations can be performed by the user by means of a single traction movement, accompanied by a gentle rotation, but without the need for multiple rotations of the device for removing the needle, as occurs with the device disclosed in US5868711. The risk of injury in the tissue of the patient during the removal process is therefore significantly reduced.

More specifically, the object of the present invention generally concerns a device for the removal of an intraosseous needle, said needle preferably being of the type comprising a Luer-Lock connector, and comprising a traction handle for the removal of the device from the body of a patient.

Throughout the present document, the expression "traction handle" will be used to designate any control mechanism adapted or configured for removing the intraosseous needle by means of the user performing a traction action, such that said traction causes the device for removal to move in the direction substantially parallel to the axis of the intraosseous needle, moving it away from the body of the patient.

Advantageously, said traction handle comprises a gripping part and a shank disposed in a substantially T-shaped arrangement, wherein a distal end of said shank comprises a connector complementary to the Luer-Lock connector arranged in the intraosseous needle.

It is thereby achieved that the method for the removal of the needle is simpler simple for health care professionals, providing an ergonomic device specially designed for exerting rotation and traction forces, but always controlled by the user of the device. Hence, during removal of the needle, the alignment of said needle with the device for removal is facilitated, such that oscillations of the needle are minimized and less damage therefore occurs in the tissues and the bone of the patient.

In a preferred embodiment of the invention, the complementary connector is a Luer-Lock threaded hole.

In a preferred embodiment of the invention, the material of the device comprises polypropylene.

The invention is therefore provided made of a rigid material capable of withstanding the required torsional stresses.

In the invention, the end of the shank opposite the handle comprises an extendible protective sleeve substantially concentric with the complementary connector housed in the shank.

Risks in the case of accidental punctures of medical staff with the needle once it has been removed are minimized by means of this design.

In a preferred embodiment of the invention, the distal end of the shank comprises a surface adapted for being placed on the skin surface of the body of a patient without causing local damage or rashes in the area surrounding the puncture point. It is an elastic element that conserves the integrity of the patient's skin without causing accidental rashes.

Better support of the device and better alignment of the shank with the needle is enabled as a result.

Likewise, and preferably, the shank of the device comprises at least one side wall, an inner cavity concentric to said wall, and a longitudinal opening from the inner cavity through the side wall. It is thereby possible to act on the protective sleeve from outside the device, in a safe manner and minimizing the risk of contamination or puncture of the user during the removal operation.

More particularly, the device comprises a slide-type button mechanically linked to the protective sleeve, so that it can be manipulated from outside the device.

According to another aspect of the invention, the protective sleeve comprises a housing for an elastic element at its end distal to the gripping part. A damping feature relating to the contact of the protective sleeve and further minimization of the risk of sustaining damage are thereby offered.

Additionally, the protective sleeve comprises a first fixing element and the slide button comprises a second fixing element cooperating with the first fixing element, such that the slide-type button is fixed to the protective sleeve. It is through said slide button that the protective sleeve can be operated, extending it or retracting it, although once the needle has been removed, the protective sleeve will be locked in its fully extended state, to prevent accidental punctures.

In a preferred embodiment of the invention, the slide-type button comprises a ridged area so that it can be gripped better by the hand and fingers of health care professionals handling the device, preferably by pushing it with the thumb.

More particularly, the ridged area is located on the upper face of the slide button for easier operation and extension or retraction of the protective sleeve, depending on whether or not the device is going to be used.

According to another aspect of the invention, the longitudinal opening comprises a widening for the insertion of the slide button so that it can be assembled and fixed on the protective sleeve, specifically, and preferably the first fixing element with the second fixing element.

According to another aspect of the invention, the longitudinal opening comprises a first groove element intended for facilitating and orienting the sliding of the slide button.

Complementarily, the slide-type button comprises a second groove element, wherein the first groove element cooperates with said second groove element such that the slide-type button can slide along the longitudinal opening of the shank and the protective sleeve can slide along the longitudinal inner cavity.

According to a first function of the invention, the protective sleeve can move from a first retracted position, in which the protective sleeve is substantially concealed in the longitudinal inner cavity, to a second extended position, in which the protective sleeve covers the intraosseous needle.

According to a second function of the invention, the protective sleeve can move from a second extended position, in which the protective sleeve covers the intraosseous needle, to a first retracted position, in which the protective sleeve is substantially concealed in the longitudinal inner cavity.

### DESCRIPTION OF THE FIGURES

Figure 1 shows a device for the removal of an intraosseous needle,
Figure 2A shows a respectively exploded perspective view of a device for the removal of an intraosseous needle according to a preferred embodiment of the present invention.
Figure 2B shows a perspective view of a device for the removal of an intraosseous needle, where the protective sleeve has a first retracted position, according to a preferred embodiment of the present invention.
Figure 2C shows a perspective view of a device for the removal of an intraosseous needle, where the protective sleeve has a second extended position, according to a preferred embodiment of the present invention.
Figure 3A specifically shows a plan view of a device for the removal of an intraosseous needle according to a preferred embodiment of the present invention.

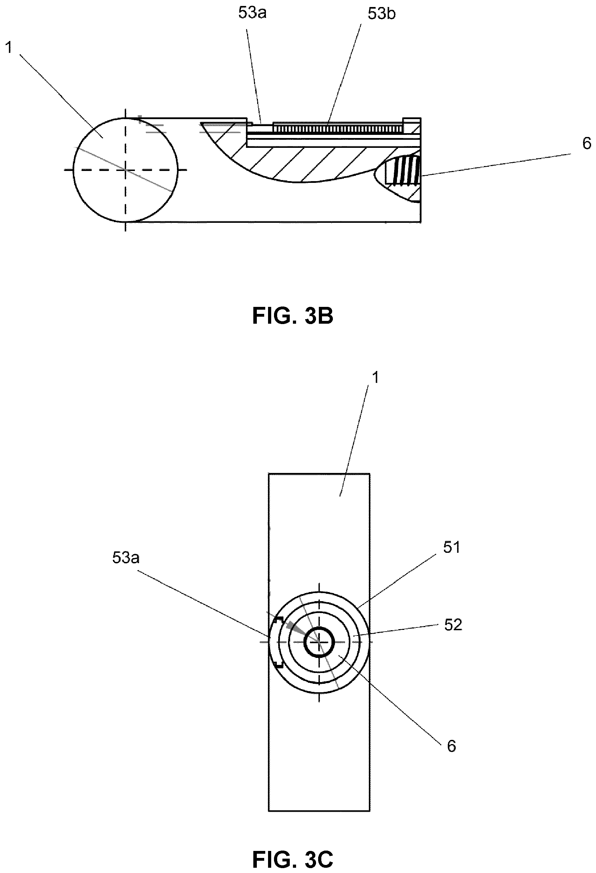

Figure 3B specifically shows a profile view of a device for the removal of an intraosseous needle according to a preferred embodiment of the present invention.
Figure 3C specifically shows a cross-section view of a device for the removal of an intraosseous needle according to a preferred embodiment of the present invention.

### REFERENCE NUMBERS USED IN THE FIGURES

For the purpose of helping to better understand the technical features of the invention, the mentioned figures are accompanied by a series of reference numbers, where the following has been depicted in an illustrative and non-limiting manner:
- (1): intraosseous needle
- (2): Luer-Lock connector
- (3): traction handle
- (4): gripping part
- (5): shank
- (51): side wall
- (52): inner cavity
- (53): opening
- (53a): widening
- (53b): first groove element
- (54): protective sleeve
- (54a): first fixing element
- (54b): housing
- (55: elastic element
- (56): slide button
- (56a): second fixing element
- (56b): second groove element
- (56c): ridged area
- (56d): upper face
- (6): complementary connector
- (7): surface at the distal end of the shank
- (81): first retracted position
- (82): second extended position

### DETAILED DESCRIPTION OF THE INVENTION

The invention and preferred embodiments are described below.

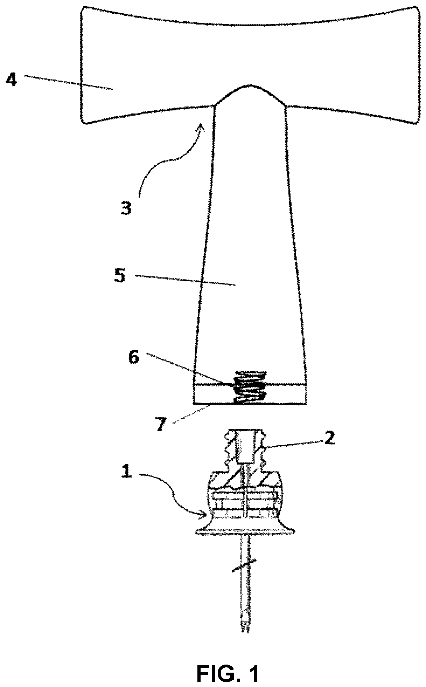

The main object of the invention concerns, as shown in Figure 1 and based on that described in the preceding sections, a specialized device for the method of removing an intraosseous needle (1) implanted in a bone of a patient. Generally, for the definition of the invention, intraosseous needles (1) known in the state of the art, which comprise a Luer-Lock type connector (2) disposed on the needle (1), shall be taken into consideration.

In turn, the device for removal comprises a traction handle (3), as defined above in the present invention, wherein said traction handle (3) comprises a gripping part (4) coupled to a shank (5) disposed in a substantially T-shaped arrangement. Likewise, the distal end of the shank (5) comprises a connector (6) complementary to the Luer-Lock connector (2) disposed on the intraosseous needle (1), such that the forces exercised during removal are transmitted by means of this connection.

By means of this configuration of the traction handle (3), where the shank (5) is centered and substantially perpendicular to the gripping part (4), an ergonomic device specially designed for generating rotation and traction forces for the purpose of removing the intraosseous needle (1) can be provided. The alignment of the needle (1) with the traction handle (3) is therefore facilitated during said removal, such that the needle (1) is prevented from bending and damaging both tissues and bone close to the area of insertion of the intraosseous needle (1).

For removal of the inserted intraosseous needle (1), the shank (5) of the device is positioned substantially aligned with the needle (1) such that the Luer-Lock connector (2) of the needle (1) can be coupled with the complementary connector (6) arranged on the shank (5). In this way, once the shank (5) is correctly aligned and the device is connected to the needle (1), when rotation and traction forces are exercised on the traction handle (3), removal of said needle (1) is achieved, preventing bending therein and, hence, minimizing damage in tissues near the area of insertion of the needle (1). The device for removal must be able to withstand the traction and torsional stresses to which the device is subjected during removal. In a preferred embodiment of the invention, the device for the removal of intraosseous needles (1) is made up of polypropylene.

In another even more preferred embodiment of the invention, both the Luer-Lock connector (2) and the complementary connector (6) arranged on the shank (5) of the device are threaded connectors.

Additionally, the invention has protective measures for the use of sharp health care instruments (biosafety elements in work equipment for accidents with a biological risk) against possible undesired punctures that may occur during the method of removal of the inserted intraosseous needle (1). To that end, the invention comprises a protective sleeve (54) arranged on the distal end of the shank (5) which is deployed at the same time the needle (1) is removed. The intraosseous needle (1) is thereby protected and contained inside the protective sleeve (54) once it has been removed, minimizing the risks of accidental punctures. As such, the invention is also, but not exclusively, comprised as a biosafety product as provided under the definitions adopted by the CDC (USA), FDA (USA), NIOSH (USA), OSHA (USA), GERES (France), and SIROH (Italy), those health care apparatus, instruments, or materials incorporating protective safety systems and designed for the purpose of eliminating or minimizing risks of exposure to accidental injuries and contagion derived from the use of sharp objects are known as biosafety devices.

The object of the invention would be comprised within an active safety device where, depending on the arrangement and design of the protective sleeve, it can be used with semi-automatic activation elements which raise the safety to passive, that is, without the need for intervention of the professional.

In a preferred embodiment, the distal surface (7) of the shank (5) is adapted for being placed on the body of the patient. Stability during the movements performed during the removal of the needle (1) is thereby favored, preventing said intraosseous needle (1) from bending and minimizing damage to surrounding tissues.

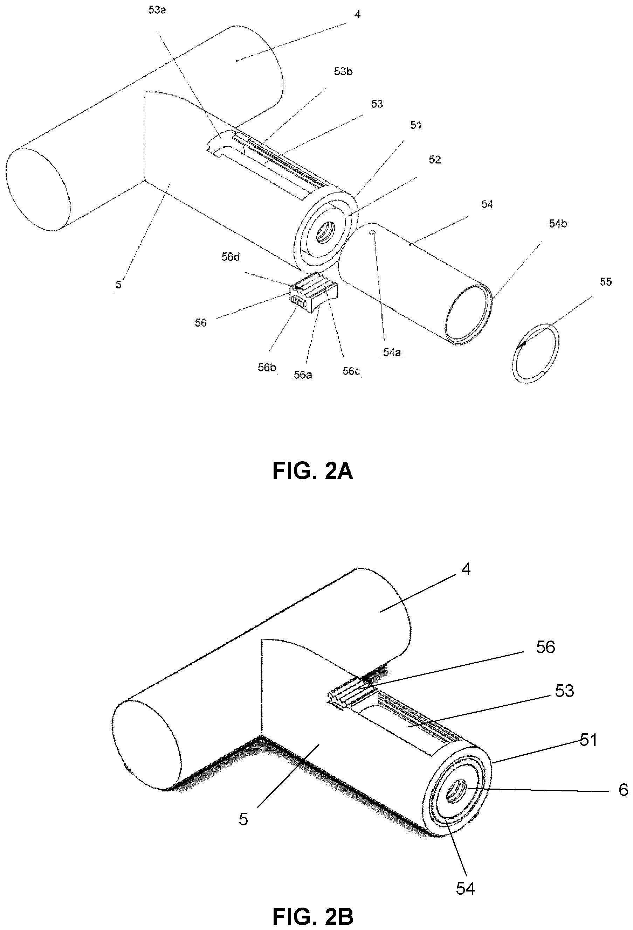

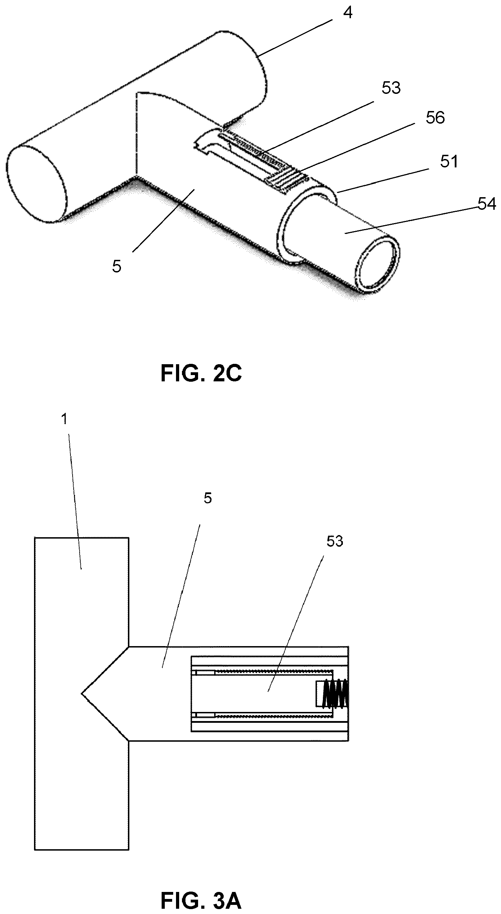

Thus and also as observed in Figure 2A *et seq.,* the device for the removal of an intraosseous needle (1), said needle (1) being of the type comprising a Luer-Lock connector (2), comprises a traction handle (3), said traction handle (3) comprises a gripping part (4) and a shank (5) disposed in a substantially T-shaped arrangement, where a distal end of said shank (5) comprises a connector (6) complementary to the Luer-Lock connector (2) arranged in the intraosseous needle (1), where the end of the shank (5) opposite the handle (3) comprises a protective sleeve (54) extendible from a longitudinal inner cavity (52) in the shank (5), wherein the protective sleeve (54) is substantially concentric with the connector (6) complementary housed in the shank (5). Moreover, the shank (5) comprises at least one side wall (51), a concentric inner cavity (52), and a longitudinal opening (53) from the longitudinal inner cavity (52) through the side wall (51).

More particularly, the device comprises a slide-type button (56) mechanically linked to the protective sleeve (54).

Additionally, the protective sleeve (54) comprises a housing (54b) for an elastic element (55) at its end distal to the gripping part (4). Said elastic element (55) is preferably a rubber washer.

According to another aspect of the invention, the protective sleeve (54) comprises a first fixing element (54a) and the slide-type button (56) comprises a second fixing element (56a) cooperating with the first fixing element (54a), such that the slide-type button (56) is fixed to the protective sleeve (54). In a preferred manner, the first fixing element (54a) is a hole, and the second fixing element (56a) is a protrusion.

More particularly, the slide-type button (56) comprises a ridged area (56c).

In a particular embodiment, the ridged area (56c) is located on the upper face (56d) of the slide button (56).

More specifically, the longitudinal opening (53) comprises a widening (53a) for the insertion of the slide button (56).

Moreover, the longitudinal opening (53) comprises a first groove element (53b).

According to a preferred embodiment of the invention, the slide-type button (56) comprises a second groove element (56b), wherein the first groove element (53b) cooperates with said second groove element (56b) such that the slide-type button (56) can slide along the longitudinal opening (53) of the shank (5) and the protective sleeve (54) can slide along the longitudinal inner cavity (52).

According to a first function of the device of the invention, the protective sleeve (54) can move from a first retracted position (81), in which the protective sleeve (54) is substantially concealed in the longitudinal inner cavity (52), to a second extended position (82), in which the protective sleeve (54) covers the intraosseous needle (1).

According to a second function of the device of the invention, the protective sleeve (54) can move from a second extended position (82), in which the protective sleeve (54) covers the intraosseous needle (1), to a first retracted position (81), in which the protective sleeve (54) is substantially concealed in the longitudinal inner cavity (52).

## Claims

1. A device for the removal of an intraosseous needle (1), said needle (1) being of the type comprising a Luer-Lock connector (2), where the device comprises a traction handle (3), and said traction handle (3) comprises a gripping part (4) and a shank (5) disposed in a substantially T-shaped arrangement, where a distal end of said shank (5) comprises a connector (6) complementary to the Luer-Lock connector (2) disposed in the intraosseous needle (1),
the device **characterized in that** the end of the shank (5) opposite the handle (3) comprises a protective sleeve (54), extendible from a longitudinal inner cavity (52) in the shank (5), wherein the protective sleeve (54) is substantially concentric with the complementary connector (6) housed in the shank (5).

2. The device according to the preceding claim, wherein the complementary connector (6) comprises a Luer-Lock threaded hole.

3. The device according to any of the preceding claims, wherein the material of the device comprises polypropylene.

4. The device according to any of the preceding claims, wherein the distal end of the shank (5) comprises a surface (7) adapted for being placed on the body of a patient.

5. The device according to any of the preceding claims, wherein the shank (5) comprises at least one side wall (51), a concentric inner cavity (52) and a longitudinal opening (53) from the longitudinal inner cavity (52) through the side wall (51).

6. The device according to claim 5, comprising a slide-type button (56) mechanically adapted for the deployment and the withdrawal of the protective sleeve (54).

7. The device according to claim 6, wherein the protective sleeve (54) comprises a housing (54b) for an elastic element (55) at its end distal to the gripping part (4).

8. The device according to claim 6, wherein the protective sleeve (54) comprises a first fixing element (54a) and the slide-type button (56) comprises a second fixing element (56a) cooperating with the first fixing element (54a), such that the slide-type button (56) is fixed to the protective sleeve (54).

9. The device according to claim 8, wherein the slide-type button (56) comprises a ridged area (56c).

10. The device according to claim 9, wherein the ridged area (56c) is located on the upper face (56d) of the slide button (56).

11. The device according to claim 8, wherein the longitudinal opening (53) comprises a widening (53a) for the insertion of the slide button (56).

12. The device according to claim 9, wherein the longitudinal opening (53) comprises a first groove element (53b).

13. The device according to claim 10, wherein the slide-type button (56) comprises a second groove element (56b), wherein the first groove element (53b) cooperates with said second groove element (56b) such that the slide-type button (56) can slide along the longitudinal opening (53) of the shank (5) and the protective sleeve (54) can slide along the longitudinal inner cavity (52).

14. The device according to any of claims 5 to 13, wherein the protective sleeve (54) can move from a first retracted position (81), in which the protective sleeve (54) is substantially concealed in the longitudinal inner cavity (52), to a second extended position (82), in which the protective sleeve (54) covers the intraosseous needle (1).

15. The device according to any of claims 5 to 14, wherein the protective sleeve (54) can move from a second extended position (82), in which the protective sleeve (54) covers the intraosseous needle (1), to a first retracted position (81), in which the protective sleeve (54) is substantially concealed in the longitudinal inner cavity (52).

## Patentansprüche

1. Vorrichtung zum Entfernen einer intraossären Nadel (1), wobei die Nadel (1) von der Art ist, die einen Luer-Lock-Anschluss (2) umfasst, wobei die Vorrichtung einen Zuggriff (3) umfasst und der Zuggriff (3) einen Griffteil (4) und einen im Wesentlichen T-förmig angeordneten Schaft (5) umfasst, wobei ein distales Ende des Schafts (5) einen in der intraossären Nadel (1) angeordneten, zum Luer-Lock-Anschluss (2) komplementären Anschluss (6) umfasst,
wobei das Gerät **dadurch gekennzeichnet ist, dass** das dem Griff (3) gegenüberliegende Ende des Schafts (5) eine Schutzhülse (54) umfasst, die aus einem längs verlaufenden Innenhohlraum (52) in dem Schaft (5) ausfahrbar ist, wobei die Schutzhülse (54) mit dem in dem Schaft (5) untergebrachten komplementären Anschluss (6) im Wesentlichen konzentrisch ist.

2. Vorrichtung gemäß dem vorhergehenden Anspruch, wobei der komplementäre Anschluss (6) ein Luer-Lock-Gewindeloch umfasst.

3. Vorrichtung gemäß einem der vorhergehenden Ansprüche, wobei das Material der Vorrichtung Polypropylen umfasst.

4. Vorrichtung gemäß einem der vorhergehenden Ansprüche, wobei das distale Ende des Schafts (5) eine Oberfläche (7) umfasst, die eingerichtet ist, um auf dem Körper eines Patienten platziert zu werden.

5. Vorrichtung gemäß einem der vorhergehenden Ansprüche, wobei der Schaft (5) mindestens eine Seitenwand (51), einen konzentrischen Innenhohlraum (52) und eine Längsöffnung (53) des längs verlaufenden Innenhohlraums (52) durch die Seitenwand (51) umfasst.

6. Vorrichtung gemäß Anspruch 5, umfassend einen Schiebeknopf (56), der mechanisch zum Ausfahren und Zurückziehen der Schutzhülse (54) eingerichtet ist.

7. Vorrichtung gemäß Anspruch 6, wobei die Schutzhülse (54) an ihrem zum Griffteil (4) distalen Ende ein Gehäuse (54b) für ein elastisches Element (55) umfasst.

8. Vorrichtung gemäß Anspruch 6, wobei die Schutzhülse (54) ein erstes Befestigungselement (54a) umfasst und der Schiebeknopf (56) ein zweites Befestigungselement (56a), das mit dem ersten Befestigungselement (54a) zusammenwirkt, umfasst, sodass der Schiebeknopf (56) an der Schutzhülse (54) befestigt ist.

9. Vorrichtung gemäß Anspruch 8, wobei der Schiebeknopf (56) einen geriffelten Bereich (56c) umfasst.

10. Vorrichtung gemäß Anspruch 9, wobei sich der geriffelte Bereich (56c) auf der Oberseite (56d) des Schiebeknopfs (56) befindet.

11. Vorrichtung gemäß Anspruch 8, wobei die Längsöffnung (53) eine Verbreiterung (53a) zum Einführen des Schiebeknopfs (56) umfasst.

12. Vorrichtung gemäß Anspruch 9, wobei die Längsöffnung (53) ein erstes Nutelement (53b) umfasst.

13. Vorrichtung gemäß Anspruch 10, wobei der Schiebeknopf (56) ein zweites Nutelement (56b) umfasst, wobei das erste Nutelement (53b) mit dem zweiten Nutelement (56b) zusammenwirkt, sodass der Schiebeknopf (56) entlang der Längsöffnung (53) des Schafts (5) und die Schutzhülse (54) entlang des längs verlaufenden Innenhohlraums (52) gleiten kann.

14. Vorrichtung gemäß einem der Ansprüche 5 bis 13, wobei sich die Schutzhülse (54) von einer ersten zurückgezogenen Position (81), in der die Schutzhülse (54) im Wesentlichen in dem längs verlaufenden Innenhohlraum (52) verborgen ist, in eine zweite ausgefahrene Position (82), in der die Schutzhülse (54) die intraossäre Nadel (1) bedeckt, bewegen kann.

15. Vorrichtung gemäß einem der Ansprüche 5 bis 14, wobei sich die Schutzhülse (54) von einer zweiten ausgefahrenen Position (82), in der die Schutzhülse (54) die intraossäre Nadel (1) bedeckt, in eine erste zurückgezogene Position (81), in der die Schutzhülse (54) im Wesentlichen in dem längs verlaufenden Innenhohlraum (52) verborgen ist, bewegen kann.

## Revendications

1. Dispositif pour le retrait d'une aiguille intra-osseuse (1), ladite aiguille (1) étant du type comprenant un connecteur Luer-Lock (2), où le dispositif comprend une poignée de traction (3), et ladite poignée de traction (3) comprend une partie de préhension (4) et une tige (5) disposées selon un agencement sensiblement en forme de T, où une extrémité distale de ladite tige (5) comprend un connecteur (6) complémentaire du connecteur Luer-Lock (2) disposé dans l'aiguille intra-osseuse (1),
le dispositif **caractérisé en ce que** l'extrémité de la tige (5) opposée a la poignée (3) comprend un manchon de protection (54), extensible depuis une cavité interne longitudinale (52) dans la tige (5), dans lequel le manchon de protection (54) est sensiblement concentrique avec le connecteur complémentaire (6) logé dans la tige (5).

2. Dispositif selon la revendication précédente, dans lequel le connecteur complémentaire (6) comprend un trou fileté Luer-Lock.

3. Dispositif selon l'une quelconque des revendications précédentes, dans lequel le matériau du dispositif comprend du polypropylène.

4. Dispositif selon l'une quelconque des revendications précédentes, dans lequel l'extrémité distale de la tige (5) comprend une surface (7) adaptée pour être placée sur le corps d'un patient.

5. Dispositif selon l'une quelconque des revendications précédentes, dans lequel la tige (5) comprend au moins une paroi latérale (51), une cavité interne concentrique (52) et une ouverture longitudinale (53) depuis la cavité interne longitudinale (52) à travers la paroi latérale (51).

6. Dispositif selon la revendication 5, comprenant un bouton coulissant (56) adapté mécaniquement pour le déploiement et le retrait du manchon de protection (54).

7. Dispositif selon la revendication 6, dans lequel le manchon de protection (54) comprend un logement (54b) pour un élément élastique (55) au niveau de son extrémité distale par rapport à la partie de préhension (4).

8. Dispositif selon la revendication 6, dans lequel le manchon de protection (54) comprend un premier élément de fixation (54a) et le bouton de type coulissant (56) comprend un second élément de fixation (56a) coopérant avec le premier élément de fixation (54a), de sorte que le bouton de type coulissant (56) est fixé au manchon de protection (54).

9. Dispositif selon la revendication 8, dans lequel le bouton de type coulissant (56) comprend une zone striée (56c).

10. Dispositif selon la revendication 9, dans lequel la zone striée (56c) est située sur la face supérieure (56d) du bouton coulissant (56).

11. Dispositif selon la revendication 8, dans lequel l'ouverture longitudinale (53) comprend un élargissement (53a) pour l'insertion du bouton coulissant (56).

12. Dispositif selon la revendication 9, dans lequel l'ouverture longitudinale (53) comprend un premier élément de rainure (53b).

13. Dispositif selon la revendication 10, dans lequel le bouton de type coulissant (56) comprend un second élément de rainure (56b), dans lequel le premier élément de rainure (53b) coopère avec ledit second élément de rainure (56b) de sorte que le bouton de type coulissant (56) peut coulisser le long de l'ouverture longitudinale (53) de la tige (5) et le manchon de protection (54) peut coulisser le long de la cavité interne longitudinale (52).

14. Dispositif selon l'une quelconque des revendications 5 à 13, dans lequel le manchon de protection (54) peut se déplacer d'une première position rétractée (81), à laquelle le manchon de protection (54) est sensiblement caché dans la cavité interne longitudinale (52), à une seconde position étendue (82), à laquelle le manchon de protection (54) recouvre l'aiguille intra-osseuse (1).

15. Dispositif selon l'une quelconque des revendications 5 à 14, dans lequel le manchon de protection (54) peut se déplacer d'une seconde position étendue (82), à laquelle le manchon de protection (54) recouvre l'aiguille intra-osseuse (1), à une première position rétractée (81), à laquelle le manchon de protection (54) est sensiblement caché dans la cavité interne longitudinale (52).
